# EUROPEAN PATENT SPECIFICATION

(11) **EP 4 531 693 B1**
(45) Date of publication and mention of the grant of the patent: **13.08.2025**
(21) Application number: 23729722.1
(22) Date of filing: 29.05.2023
(51) Int. Cl.: A61B 8/00

(54) **GENERATION OF ULTRASOUND SELF-SCAN INSTRUCTIONAL VIDEO**
ERZEUGUNG VON ULTRASCHALL-SELBSTABTAST-ANLEITUNGSVIDEO
GÉNÉRATION DE VIDÉO D'INSTRUCTIONS POUR AUTO-ÉCHOGRAPHIE

(30) Priority: 31.05.2022 US 202263347112 P
(43) Date of publication of application: 09.04.2025
(73) Proprietor: Koninklijke Philips N.V., 5656 AG Eindhoven (NL)
(72) Inventor: PREVRHAL, Sven Peter, 5656 AG Eindhoven (NL); ERRICO, Claudia, 5656 AG Eindhoven (NL); BHARAT, Shyam, 5656 AG Eindhoven (NL)
(74) Representative: Philips Intellectual Property & Standards
(86) International application number: PCT/EP2023/064306
(87) International publication number: WO 2023/232730

(56) References cited:
- US-A1- 2010 179 428
- US-A1- 2019 125 301
- US-A1- 2021 327 304

## Description

### FIELD OF THE INVENTION

The invention relates to the field of ultrasound imaging and more particularly to generation of ultrasound self-scan instructional video for follow-up diagnostic ultrasound imaging by a user who is not a skilled sonographer.

### BACKGROUND

With the evolution of medical diagnosis toward support by objectively and metrically captured evidence, a growing demand and reliance is placed on technical assistance to doctors, especially on medical imaging modalities. The numeric increase of medical imaging exams is facing steepening challenges, firstly with respect to availability of operating staff and doctors skilled enough to perform the exam and interpret its results. Secondly, despite imaging technical kits being almost fully commoditized, financial pressure increases with respect to the considerable labor cost of care delivery.

This has led to deferring many tasks once strictly performed in high professional care to less expensive settings, from wound dressing at home by skilled workers to remote reading of radiology images in lower-cost countries. In ultrasound imaging the challenge in using unskilled workers has traditionally been the high level of knowledge and expertise needed to acquire and interpret the image.

US 2021/327304 A1 discloses guiding a user performing a medical procedure with an ultrasound probe. It is disclosed that a reference performance carried out by a proficient user before the medical procedure by the user is recorded. This reference procedure can be considered to be an initial ultrasound imaging scan. During the medical procedure by the user, the movement of the probe is compared to the reference procedure and position-based feedback guidance in the form of instructions to correct the users performance is displayed in real-time.

### SUMMARY

Carrying out medical diagnostic exams in non-professional environments and in the absence of professional care personnel is increasingly being explored, for instance in at-home or tertiary care and rehabilitation centers. Extremely portable ultrasound imaging hardware now exists that technically and logistically supports "self-scanning" and similar use scenarios. However, for this modality, the high level of knowledge and expertise needed to acquire and interpret the images is a barrier to use in non-professional environments. There are considerable challenges to obtain images with satisfactory diagnostic value when the scan is performed by an unskilled user (e.g., the patient themselves or a family member etc.). The present inventors have determined that (a) on the journey to making lay-person administration of an ultrasound exam a reality, detailed, task- and patient-specific training will be essential, (b) personalized video instruction is a preferred means to administer such training and (c) creation of such content should be cost- and time-efficient and minimize interference with a sonographer's routine professional tasks. The present invention provides a method for automatically generating patient-specific training video material for the patient for self-scanning.

While all other medical imaging equipment still carries a footprint too expensive and/or large and immobile for use outside of clinics and doctor's offices, for ultrasound (US) imaging, extremely portable hardware now exists that technically and logistically supports "self-scanning" and similar use scenarios. Philips' Lumify^{®} is a perfect representation of this trend.

The smallness of the Lumify^{®} product would support increased production, and potentially concomitant decreasing per-sale margin. Higher technical capacity for ultrasound exams in healthcare might lead to better care, for one by enabling more frequent follow-up imaging exams, if the bottleneck of the staff shortage this would create could be circumnavigated. One way to accomplish this is to widen the circles of people who can administer an ultrasound exam, by lifting more people up to the needed skills standards or lowering standards, or a combination of both.

The present invention widens the circle of people who can administer an ultrasound exam with an ultramobile device such as Lumify^{®} to include lay people, including the patients themselves, by providing an automatically generated, personalized, i.e. task-specific and patient-specific instructional video that summarizes the most critical steps of the expert's scan. The present invention eliminates the need for an unskilled person administering an ultrasound scan to view and interpret ultrasound images, a task that requires significant skill.

In the present invention, an initial ultrasound imaging scan in a clinical environment with a skilled sonographer is used to generate an ultrasound self-scan instructional video. Using the self-scan instructional video an unskilled user does not need to evaluate images and can simply copy the clinical procedure to perform a follow-up ultrasound imaging scan in a non-clinical environment. Indeed, there may be no need to show the user/patient actual ultrasound images obtained during the procedure, because the unskilled user cannot accurately interpret the images. Thus, the instructional video may only show how to (self-) administer the ultrasound scan (i.e., the movements and settings) without actually displaying the scan image.

According to a first aspect of the present invention, a method is provided for generating an ultrasound self-scan video. The method comprises, capturing a landmark location, tracking data for an ultrasound probe with timestamps, and user interactions with timestamps, during an initial ultrasound imaging scan, wherein the user interactions comprise identification of a keyframe. The method further comprises using the landmark location, the tracking data, the identified key frame, and the timestamps, to plan a probe movement path from the landmark to an ultrasound probe location corresponding to the keyframe. After planning the probe movement path, an ultrasound self-scan instruction video is generated showing the landmark and planned probe movement path to the probe location corresponding to the key frame.

According to an exemplary embodiment of the present invention, the user interactions further comprise ultrasound probe settings, and the self-scan training video further indicates the ultrasound probe settings.

According to an exemplary embodiment of the present invention, capturing a landmark location and tracking data for an ultrasound probe comprises recording video of the initial ultrasound imaging scan and extracting the landmark location and ultrasound probe position data from the video.

According to an exemplary embodiment of the present invention, capturing user interactions comprises extracting user interaction data from a log file.

According to an exemplary embodiment of the present invention, capturing user interactions comprises recording video of a user interface and extracting user interactions from the video.

According to an exemplary embodiment of the present invention, the step of generating the ultrasound self-scan training video comprises video images recorded during the initial ultrasound imaging scan.

According to an exemplary embodiment of the present invention the video images are edited to show the ultrasound probe following the planned path from the landmark to the location corresponding to the keyframe.

According to an exemplary embodiment of the present invention: a key zone is identified around each location corresponding to the keyframe to compensate for tracking inaccuracies; and the step of generating the ultrasound self-scan training video comprises creating an animation of an ultrasound probe following the planned path from the landmark to the location corresponding to the keyframe.

According to an exemplary embodiment of the present invention the step of generating the ultrasound self-scan training video comprises graphical or auditory instructions to duplicate the probe settings.

According to an exemplary embodiment of the present invention the step of generating the ultrasound self-scan training video further comprises graphical or auditory instructions to make one or more practice runs of the planned path while the ultrasound scanner is turned off, prior to performing an ultrasound self-scan with the ultrasound scanner turned on.

A proposed use of the instructional video is that the user is encouraged to not only watch it but to practice several dry runs with the equipment in hand and position, following the instructions as closely as possible, for a final run with the US equipment actually turned on. Therefore. according to an exemplary embodiment, the step of generating the ultrasound self-scan instructional video further comprises: configuring the ultrasound self-scan instructional video to obtain tracking data for a self-scan ultrasound probe; and overlaying a position indicator for the self-scan probe on the self-scan ultrasound instructional video to provide feedback to the unskilled user.

According to second aspect of the present invention a computer program product is provided, comprising a machine -readable media having encoded thereon, a machine-executable program of instructions, which when executed by a processor, performs the steps of:
during an initial ultrasound imaging scan, capturing a landmark location, tracking data for an ultrasound probe with timestamps, and user interactions with timestamps, wherein the user interactions comprise identification of a keyframe;
using the landmark location, the tracking data, the identified key frame, and the timestamps, planning a path from the landmark to an ultrasound probe location corresponding to the keyframe;
generating an ultrasound self-scan instruction video showing the landmark and planned path to the probe location corresponding to the key frame.

According to a third aspect of the present invention a system for generating an ultrasound self-scan training video is provided. The system comprises a processor and a memory operably connected to the processor and having encoded thereon, computer-executable program code, configured to: receive tracking data identifying the location and orientation of an ultrasound probe relative to an anatomical landmark during an ultrasound imaging procedure; receive interaction data from a log file and identifying keyframes from the interaction data; plan a path from the landmark to a position corresponding to each keyframe; and generate the ultrasound self-scan training video showing the landmark and planned path to the location corresponding to each keyframe.

According to an exemplary embodiment of the present invention, the system further comprises: an ultrasound probe and a camera, wherein, the probe and the camera are operably connected to the processor; wherein the camera is configured to capture the locations of an anatomical landmark and the ultrasound probe during an initial scan; and wherein the computer-executable program code is further configured to: control the ultrasound probe to propagate and receive ultrasound signals and receive ultrasound image data from the ultrasound probe; and receive the video stream from the camera, identify the landmark on the video image stream, determine the location of the ultrasound probe relative to the anatomical landmark, and provide the location of the ultrasound probe relative to the anatomical landmark as tracking data.

According to an exemplary embodiment of the present invention, the system, further comprises: an ultrasound probe and a tracking sensor mounted on the ultrasound probe. The probe and the tracking sensor are operably connected to the processor. The tracking sensor is configured to generate tracking data capturing locations for an anatomical landmark and the ultrasound probe during an initial scan. The computer-executable program code is further configured to: control the ultrasound probe to propagate and receive ultrasound signals and receive from the ultrasound probe and receive tracking data from the tracking sensor, and determine the location of the ultrasound probe relative to the anatomical landmark.

When used herein, the term "keyframe" shall mean an image that defines the starting point and ending point of a transition, namely an image that provides a preferred view of a patient feature for diagnostic purposes.

When used herein the term "animation" shall mean the manipulation of electronic images by means of a computer in order to create moving images.

### BRIEF DESCRIPTION OF THE DRAWINGS

The features and advantages of the invention will be more clearly understood from the following detailed description of the preferred embodiments when read in connection with the accompanying drawing. Included in the drawing are the following figures:
Fig. 1 shows a system for generating an ultrasound self-scan instructional video according to an embodiment of the present invention;

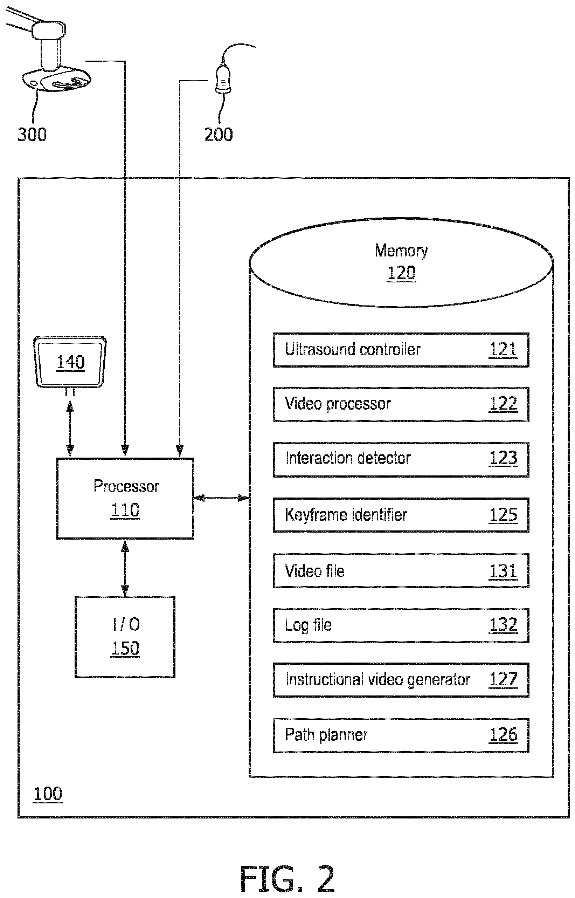

Fig. 2 is a block diagram of a system for generating an ultrasound self-scan instructional video according to an embodiment of the present invention;
Fig. 3 is a diagram showing a view of a patient with an anatomical landmark and planned ultrasound paths according to an embodiment of the present invention;
Fig. 4 is a diagram showing key zones according to an embodiment of the present invention;
Fig. 5 is a block diagram of a method for generating an ultrasound self-scan instructional video according to an embodiment of the present invention; and
Fig. 6 is a flow diagram of the method for generating an ultrasound self-scan instructional video of Fig. 5.

### DETAILED DESCRIPTION

The present invention provides a method and system for generating an ultrasound self-scan instructional video to guide an unskilled user through an ultrasound imaging procedure.

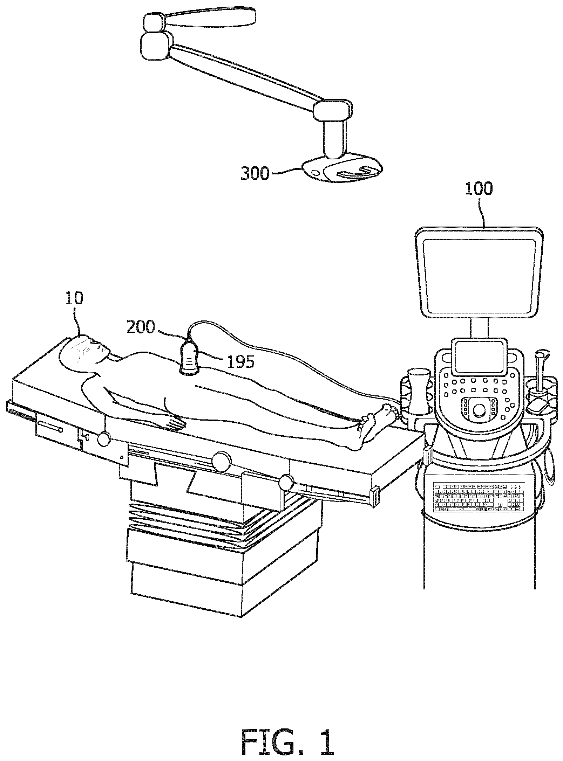

Referring to Fig. 1, according to one embodiment of the present invention, a system for automatically generating an ultrasound self-scan instructional video is shown. The system comprises an ultrasound system for use in a clinical setting to generate sonographs of a patient 10. The ultrasound system comprises an ultrasound probe 200 operably connected to an ultrasound controller 121 in computer 100.

The ultrasound probe 200 comprises one or more ultrasound transducers for propagating and receiving ultrasound signals and transmitting the received ultrasound signals to the ultrasound controller 121 in the computer 100. Computer 100 may be an ultrasound system console, and may comprise hardware circuits or executable program code in software, the computer 100 including an ultrasound controller 121. The ultrasound probe 200 may be any ultrasound probe currently commercially available or developed in the future.

The ultrasound controller 121 comprises program code executable by a processor 110 to control the ultrasound probe to propagate and receive ultrasound signals, to transmit the received signals and to process the transmitted signals to generate a sonagraph image. The ultrasound controller may be embodied in any commercially available ultrasound system or any ultrasound system developed in the future. The ultrasound probe 200 may be operably connected to the ultrasound controller 121 by an electrical cable or by wireless communication between the ultrasound probe 200 and the processor 110 executing the program code of the ultrasound controller 121.

The system for automatically generating a self-scan instructional video further comprises a tracking system for tracking the probe position during an initial ultrasound scan in a clinical setting. According to one embodiment of the invention, the tracking system is an optical tracking system comprising a camera 300 and a video processor 122. The camera may be any camera having a field of view sufficient to capture the ultrasound probe 200 and an anatomical landmark 400 and provide a stream of video data to the video processor 122. The video processor 122 comprises program code executable by a processor 110 to process the stream of video data from the camera 300 to identify and locate the anatomical landmark 400, and to determine a location of the ultrasound probe relative to the landmark with a timestamp. The anatomical landmark 400 may be any identifiable anatomical feature proximate to the area to be scanned, for example: a navel or a nipple.

The video processor 122 utilizes deep learning-based image processing to reliably and automatically determine the kinds of landmark(s) that is (are) visible in the video frames, as well as the position and orientation of the US probe per frame, using a pattern matching approach. The deep learning process can be unsupervised, using statistical analysis. For instance, optimizing landmark identification for well-recognizable optical features that are common to the training images and are points that planned probe movement paths have in common. The deep learning process can also be supervised. That is, an operator labels the landmarks in the training data.

The video processor 122 defines a patient-specific and landmark-specific image coordinate system that allows determining the probe position corresponding to the key frames relative to the landmark (in case of several landmarks, the nearest one). The patient -specific and landmark-specific image coordinate system may be defined by registering the landmark and the patient orientation. To define the orientation, the prominent body axis of patient 10 needs to be captured. In most cases, this will be parallel to the spine. The tracking camera will generally look at the scene from an oblique angle (not straight on), so there will be perspective skewing which has to be corrected for ("capturing the scale"). For capturing the scale, either the true dimensions of the probe are known or a scale (for instance simple crosshairs or 2D barcode patterns) are affixed on the probe such that at least one of them is visible to the video camera at all times. As known from augmented-reality (AR) technology, the distance and the orientation of the pattern can then be easily ascertained by AR image processing software.

According to one embodiment the video camera 300 is placed on top of an ultrasound cart housing the video controller such that the camera's field of view encompasses the area to be examined and the anatomical landmark 400. The camera may be a self-rotating camera capable of automatically following the probe movement. Alternatively, the camera angle may be adjusted by the ultrasound operator or sonographer. In another embodiment, the camera may be mounted on the sonographer' head. In yet another embodiment the camera comprises two or more spatially separated co-registered camera to avoid obscured areas in the field of view.

When the initial clinical ultrasound exam starts, the video capture also starts and begins recording video footage (images) of the sonographer's hand holding the probe and moving and tilting it across the patient's skin surface. The camera field of view is wide enough such that the video footage also includes one or more easily identifiable landmarks, e.g., the belly button or nipples. The video of the ultrasound exam provides an example for a non-skilled user to duplicate the motions of the ultrasound probe to perform a patient/diagnostic specific follow-up scan. The video camera footage is processed to extract the probe movements relative to the landmarks, including pan, tilt, and pitch movements. Then, these movements are simplified to generate the easiest movement to obtain the image at each keyframe location (the location corresponding to each diagnostically valuable image or frame), as will be explained hereafter.

In an alternate embodiment, the tracking system does not use a camera, but rather uses another modality (e.g., inertial sensors, magnetic sensors, etc). A tracking sensor 195 (inertial, magnetic, etc.) is located on the ultrasound probe used during the initial scan. Ultrasound probe mounted sensors are known in the art. The sensor location must be registered to the patient, such as by the sonographer tapping the anatomical landmark and moving the probe along the main patient axis prior to beginning the initial scan. When the sonographer taps the anatomical landmark, the position may be registered through an input/output device 150, such as a mouse, keyboard, foot pedal, microphone capturing a verbal signal, or any other appropriate input/output device.

The system for automatically generating a self-scan instructional video further comprises an interaction detector 123. The interaction detector 123 may comprise program code configured to search a log file 132 for user interactions, such as screenshots taken, ultrasound beam manipulations, and ultrasound mode changes with the event timestamps. Alternatively, the interaction detector 123 may be program code configured to intercept user inputs directly from one or more user input devices, such as a keyboard, mouse, foot pedal, and the like. The interaction detector would also acquire a timestamp when intercepting user interactions. In another alternative embodiment, the interaction detector may comprise a microphone and voice recognition program code to detect verbal indications of user interactions provided by the sonographer together with timestamps to mark user interactions. In yet another alternative embodiment, the interaction detector 123 may comprise a camera directed at controls for the ultrasound system and video processing program code to identify interactions. This may be the same camera 300 as the camera used for tracking with a field of view wide enough to also capture user interactions at user input devices or may be a separate camera.

A video processor 121 matches the time stamp of a detected screen shot with the time stamp of a probe location to identify the probe location corresponding to a key frame (the image frame at which the sonographer initiated a screen shot because of its diagnostic value).

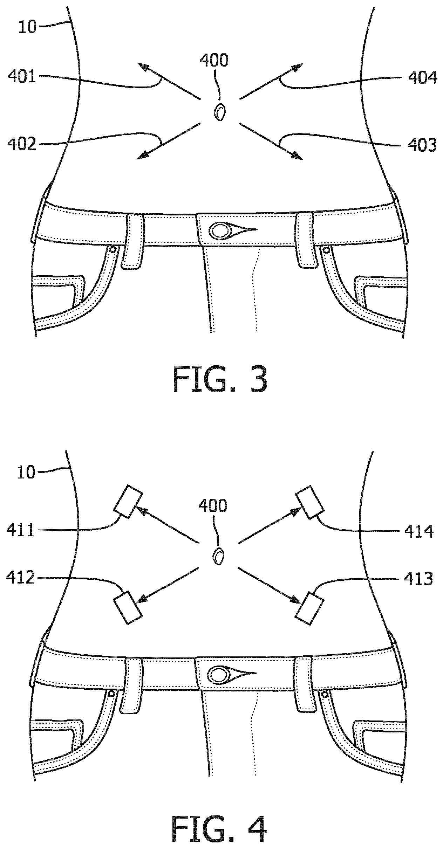

The system for automatically generating a self-scan instructional video further comprises a path planner 126. The path planner may comprise program code configured to calculate one or more direct paths 401-404 (shown in Fig. 3) from the anatomical landmark 400 to the location corresponding to each key frame.

During the initial US imaging exam, the sonographer may make probe movements and orientations while observing sonographs on a display 140 operably connected to the ultrasound probe 200 through the ultrasound controller 121 to find an image with high diagnostic value, and this process is not always straight-forward, i.e. the sonographer will often move the probe and re-orient it many times. These movements are defined as search movements, performed in search of the best position and orientation. Advantageously, in the present invention the unskilled user does not have to evaluate the diagnostic value of ultrasound images. Instead, the unskilled user simply needs to duplicate the probe position and orientation for each key frame. Therefore, during the self-scan, the patient does not need to replicate these search movements, and the probe movement and orientation path that is shown to the patient may be a simplified path that shows only those movements needed to move the probe to the keyframe(s). Since the position and orientation of each key frame is known, the system and method enables the replacement of the sonographer's movements with movements that lead straight to the location of the key frame.

In order to avoid accumulation of movement error, the ultrasound self-scan instructional video may instruct the patient or other unskilled user to always go back to a "safe spot" during key frames, i.e. the anatomical landmark 400, and start afresh from there. This creates a star-shaped movement path with the anatomical landmark at the star's center. The anatomical landmark is not bound to be located in the center of the pattern. Any easily identifiable position will do, which could lead to "lopsided" star shapes. For very simple exams, the approach can be altered to directly go from one key frame to the next.

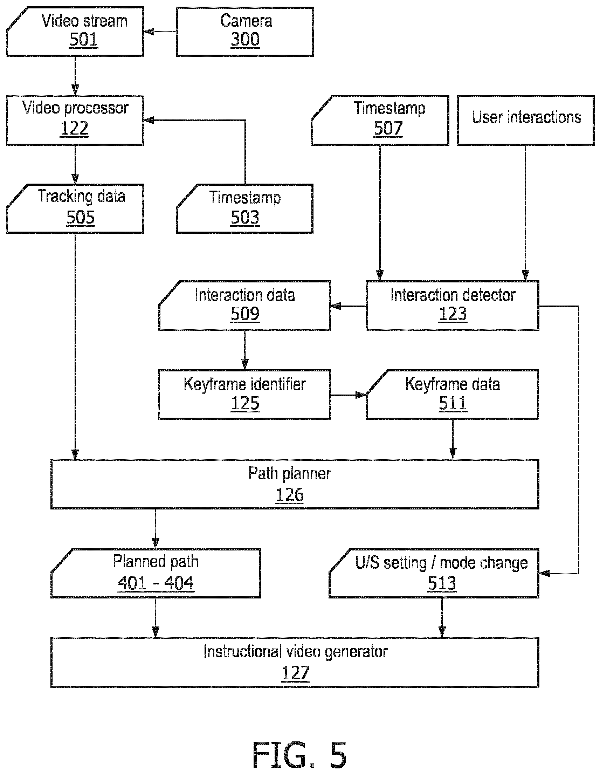

Since the patient or unskilled user may not be able to replicate the position and orientation of the key frame exactly, the position of the key frame may be extended to one or more of a key zone 411-414, as shown in Fig. 5. For example, if the key frame lies 10 cm to the left of the anatomical landmark, the key zone will extend from 7 to 13 cm. By scanning the entire key zone in increments, the system and method increase the likelihood of capturing an image of high diagnostic value.

For each key zone 411-414, the ultrasound self-scan instructional video graphically instructs the patient or scan-administering person to position the probe at the anatomical landmark, then slowly move toward the key zone. Once the edge of the key zone is reached, the instruction is to slowly tilt and rotate the probe to match the orientation the sonographer assumed during the initial scan, and then continue incrementally outward during the opposing end of the key zone. The probe orientation may be shown by the instructional video using an animated rendering derived from the tracking system showing the orientation from the perspective of the unskilled user. If the tracking system is a video feed of the initial scan, the camera angle may be significantly different from the unskilled user's perspective. Alternatively, the orientation may be shown using video from the initial scan if the video is generated by a camera positioned to replicate the unskilled user's perspective, such as being mounted on the sonographer's head or eyeglasses where the unskilled user is not the patient. The ultrasound self-scan instructional video may also use a combination of animation and video images from the initial scan. At each increment, small back-and-forth tilts and rotations are prescribed. This ensures that any inaccuracy in positioning is balanced out and an US image of high diagnostic value is captured with high probability. After each key zone coverage, the user is instructed to reposition the probe on top of the anatomical landmark, without having to retrace the path backwards.

The video processing code may be configured to extract and display a sequence of user interactions, based in part on the information obtained during the prior exam, as part of the ultrasound self-scan instructional video. That is, the video may show captured images of the user manipulating a user interface device, such as a keyboard. Alternatively, the video processing code may use pattern recognition to detect user interactions from a video stream from the camera. Timestamps would also be acquired for user interactions extracted from a camera video stream.

The system for automatically generating a self-scan instructional video further comprises an instructional video generator 127. The instructional video generator automatically generates an instructional video showing probe movements (translation and orientation) along planned paths 401-404 (shown in Fig. 3) generated by the path planner 126 going directly from the anatomical landmark 400 to each key frame location or key zone 411-414. The self-scan instructional video may use images captured by camera 300 to generate the video, editing out frames that are not in the planned path, due to the iterative nature of the clinical scan. Alternatively, the instructional video generator may generate an animation based on the planned paths 401-404, as described below.

The self-scan instructional video may further comprise directions for ultrasound scan settings, such as mode changes and beam manipulations (e.g., steering, focusing, shaping) captured by the interaction detector 123. These directions may be provided in the form of audio instructions, video of a user interface, or animations.

In one embodiment, pre-recorded oral instructions from pre-recorded voice snippets are added to the ultrasound self-scan instructional video. These instructions may provide guidance to duplicate the initial scan, such as "Slowly move the probe 5 cm left" or "Slightly tilt the probe towards you".

The ultrasound self-scan instructional video may be stored on a computer-readable medium. This medium can be an electronic, magnetic, optical, electromagnetic, infrared, or semiconductor system (or apparatus or device). Examples of a computer-readable medium include a semiconductor or solid state memory, magnetic tape, a removable computer diskette, a thumb drive, a random access memory (RAM), a read-only memory (ROM), a rigid magnetic disk, an MPEG file, and an optical disk. Current examples of optical disks include compact disk-read only memory (CD-ROM), compact disk-read/write (CD-R/W) and DVD. Alternatively, the ultrasound self-scan instructional video may be embodied in a video stream delivered over the internet or on a cable or broadcast television program, or stored on a DVD, a VHS tape.

The home user (patient or other unskilled user) loads the medium with the instructional video into an appropriate device and plays the instructional video. Depending on the medium used, the instructional video may be uploaded onto a laptop, smartphone, or tablet. The tablet may be part of the home user's ultrasound system. The home user plays the instructional video, watches, and potentially listens to the instructional video, and follows set-up instructions embedded in the instructional video. The home user executes probe movements, mimicking the instruction video which shows the planned path from the home user's point of view. Then, the home user executes completion instructions provided on the instructional video, including uploading scanned imaging data for review by a trained sonographer.

According to one embodiment, the ultrasound probe 200 is be equipped with position sensors 195, such as optical, magnetic, inertial or other known position sensors. These position sensors increase the accuracy of capturing probe position and/or orientation during key frames.

In certain embodiments, the video camera captures the video from a location/perspective that is not similar to that of the patient's perspective or the sonographer's perspective. In these embodiments, the video cannot be directly used as input to the instructional video. Instead, animated movie clips are automatically generated, showing a representation of a skin patch resembling the target scan area (e.g., the belly) and the sequence of the star-shaped probe movements and orientations from the sonographer's perspective and/or the patient's perspective. The animation may be generated using the patient-specific and landmark-specific image coordinate system and scale defined by the video processor 122. The instructional video generator 127 may generate an animation by overlaying an image of the anatomical landmark and the ultrasound probe onto a simulated skin patch at their relative positions provided in the tracking data for each video frame. An orientation reference may also be provided such as a patient's sides showing the prominent body axis or an axis line corresponding to the prominent body axis. The planned path may also be overlaid onto the video.

In one embodiment, pre-recorded oral instructions from pre-recorded voice snippets are added to the ultrasound self-scan instructional video. These instructions may provide guidance to duplicate the initial scan, such as "Slowly move the probe 5 cm left" or "Slightly tilt the probe towards you".

The instructional video can be extended by prepending pre-made video clip sequences showing how to prepare for the exam, such as preparing the US device, seating or lying down, applying coupling gel, starting the exam and after the core part, appending pre-made video clip sequences showing how to finish the exam, release the date for transmission, power down the device etc.

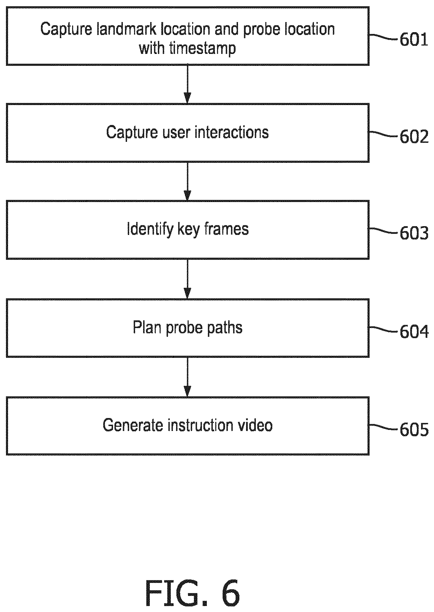

Referring to Figs. 5 and 6, a method of practicing the present invention is now described. An initial ultrasound imaging scan is performed in a clinical environment by a skilled sonographer. During the initial ultrasound scan, a tracking system captures at step 601 a landmark location and tracking data for an ultrasound probe position relative to the landmark with timestamps. According to one embodiment, the tracking system comprises a camera 300 and a video processor 122. The video processor 122 obtains a video stream 501 from the camera 300. The video processor identifies an anatomical landmark using a deep learning algorithm as described above. The video processor also identifies the ultrasound probe and calculates the probes position relative to the anatomical landmark for each video frame with a timestamp 503. The video processor 122 provides the probe position relative to the anatomical landmark with time stamp to the path planner 126 as tracking data 505.

Also during the initial ultrasound scan, the interaction detector 123 detects user interactions at step 602 and annotates the interactions with timestamps 507. The interaction detector 123 provides an interaction identification (i.e., type of interaction) and timestamp to the keyframe identifier 125 as interaction data 509. From the user interaction data, the keyframe identifier 125 identifies a key frame at step 603. The keyframe may be identified by the sonographer taking a screen shot, or by the sonographer providing an input through an input device, such as a verbal signal through a microphone, or a tactile signal through a keyboard or mouse. The keyframe data 511, timestamp of the keyframe, is provided to the path planner 126. The interaction detector 123 also provides ultrasound settings and mode changes 513 to the instructional video generator 127.

Using the landmark location, the tracking data 505, the identified key frame data 511, and the timestamps, the path planner 126 plans at step 604 a path 401-404 directly from the landmark to an ultrasound probe location corresponding to each keyframe. The path planner 126 matches probe locations from the tracking data with identified keyframes by matching the timestamps. Using the now known keyframe locations, the path planer calculates a planned path 401-404 which comprises a direct path from the anatomical landmark to each keyframe location.

In order maximize the likelihood of obtaining images of high diagnostic value, the planned path may also provide incremental translations of the probe across the key zones 411-414 and incremental orientation adjustments at each incremental step of translation, that is, the key zones may be provided to obtain denser ultrasound data capture coverage at many positions and many probe orientations.

Using the planned path and interaction data, such as mode changes and beam manipulations, the instructional video generator 127, generates an instructional video at step 605 to guide an unskilled user through an ultrasound scan. The generated ultrasound self-scan instruction video shows the landmark and planned path to and through the key zones. The planned path may be shown using animation, video captured during the initial ultrasound scan or a combination of thereof.

According to one embodiment of the present invention, the generated ultrasound self-scan instructional video further comprises instructions for the user to make one or more practice runs of the scan before turning on the ultrasound scanner to familiarize the user with the probe movements.

According to one embodiment, the ultrasound self-scan instructional video is configured to obtain tracking data for an ultrasound probe during practice runs, during the actual scan by an unskilled user, or both. Then, a position indicator may be overlayed on the ultrasound self-scan instructional video animation to provide feedback to the unskilled user showing how well the movements correspond to the desired movements on the instructional video.

The invention can take the form of an entirely hardware embodiment, an entirely software embodiment or an embodiment containing both hardware and software elements. In an exemplary embodiment, the invention is implemented in software, which includes but is not limited to firmware, resident software, microcode, etc.

Furthermore, the invention may take the form of a computer program product accessible from a computer-usable or computer-readable medium providing program code for use by or in connection with a computer or any instruction execution system or device.

The medium can be an electronic, magnetic, optical, electromagnetic, infrared, or semiconductor system (or apparatus or device) or a propagation medium. Examples of a computer-readable medium include a semiconductor or solid state memory, magnetic tape, a removable computer diskette or thumb drive, a random access memory (RAM), a read-only memory (ROM), a rigid magnetic disk, and an optical disk. Current examples of optical disks include compact disk-read only memory (CD-ROM), compact disk-read/write (CD-R/W) and DVD.

The foregoing method may be realized by a program product comprising a machine -readable media having a machine-executable program of instructions, which when executed by a machine, such as a computer, performs the steps of the method. This program product may be stored on any of a variety of known machine-readable media, including but not limited to compact discs, floppy discs, USB memory devices, and the like.

The preceding description and accompanying drawing are intended to be illustrative and not limiting of the invention. The scope of the invention is intended to encompass equivalent variations and configurations to the full extent of the following claims.

### REFERENCE NUMBER LISTING

- 10: patient
- 100: computer (workstation)
- 110: processor
- 120: memory
- 121: ultrasound controller
- 122: video processor
- 123: interaction detector
- 125: key frame identifier
- 126: path planner
- 127: instructional video generator
- 131: video file
- 132: log file
- 140: display
- 150: input/output
- 300: camera
- 200: ultrasound probe
- 195: position sensors
- 400: anatomical landmark
- 401-404: planned paths
- 411-414: key zones
- 501: video stream
- 503: video time stamp
- 505: trackingn data
- 507: ultrasound time stamp
- 509: user interaction data
- 511: key frame data
- 513: U/S settings / mode change
- 601: capture landmark & probe locations
- 602: capture user interactions
- 603: identify key frames
- 604: plan paths
- 605: generate instructional video

## Claims

1. A method for generating an ultrasound self-scan video, comprising the steps of:
during an initial ultrasound imaging scan, capturing (601, 602) a landmark (400) location and tracking data (505) for an ultrasound probe (200) with timestamps (503), and
user interactions with timestamps (507), wherein the user interactions comprise identification (603) of a keyframe (511);
using the landmark (400) location, the tracking data (505), the identified key frame (511), and the timestamps (503, 507), planning (604) a path (401-404) from the landmark (400) to an ultrasound probe (200) location corresponding to the keyframe (511); and
generating (605) the ultrasound self-scan instruction video showing the landmark (400) and planned path (401-404) to the probe (200) location corresponding to the key frame (511).

2. The method of claim 1, wherein the user interactions further comprise ultrasound probe settings, and the self-scan training video further indicates the ultrasound probe settings.

3. The method of claim 1, wherein capturing (601) a landmark (400) location and tracking data (505) for an ultrasound probe (200) comprises recording video of the initial ultrasound imaging scan and extracting the landmark location and ultrasound probe position data from the video.

4. The method of claim 1, wherein capturing (602) user interactions comprises extracting user interaction data (509) from a log file (132).

5. The method of claim 1, wherein capturing (602) user interactions comprises recording video of a user interface and extracting user interactions from the video.

6. The method of claim 1, wherein the step of generating (605) the ultrasound self-scan training video comprises video images recorded during the initial ultrasound imaging scan.

7. The method of claim 6, wherein the video images are edited to show the ultrasound probe (200) following the planned path (401-404) from the landmark (400) to the location corresponding to the keyframe (511).

8. The method of claim 1, wherein:
a key zone (411-414) is identified around each location corresponding to the keyframe (511) to compensate for tracking inaccuracies; and
the step of generating (605) the ultrasound self-scan training video comprises creating an animation of an ultrasound probe (200) following the planned path (401-404) from the landmark (400) to the location corresponding to the keyframe (511).

9. The method of claim 2, wherein the step of generating (605) the ultrasound self-scan training video comprises graphical or auditory instructions to duplicate the probe settings.

10. The method of claim 9, wherein the step of generating (605) the ultrasound self-scan training video further comprises providing graphical or auditory instructions to make one or more practice runs of the planned path (401-404) while the ultrasound scanner is turned off, prior to performing an ultrasound self-scan with the ultrasound scanner turned on.

11. The method of claim 10, wherein the step of generating (605) the ultrasound self-scan instructional video further comprises:
configuring the ultrasound self-scan instructional video to obtain tracking data for a self-scan ultrasound probe; and
overlaying a position indicator for the self-scan probe on the self-scan ultrasound instructional video to provide feedback to the unskilled user.

12. A computer program product comprising a machine -readable media having encoded thereon, a machine-executable program of instructions, which when executed by a processor (110), performs the steps of:
during an initial ultrasound imaging scan, capturing (601, 602) a landmark (400) location, tracking data (505) for an ultrasound probe (200) with timestamps (503), and user interactions with timestamps (507), wherein the user interactions comprise identification (603) of a keyframe (511);
using the landmark (400) location, the tracking data (505), the identified key frame (511), and the timestamps (503, 507), planning (604) a path (401-404) from the landmark (400) to an ultrasound probe (200) location corresponding to the keyframe (511); and
generating (605) an ultrasound self-scan instruction video showing the landmark (400) and planned path (401-404) to the probe (200) location corresponding to the key frame (511).

13. A system (100) for generating an ultrasound self-scan training video, comprising:
- a processor (110); and
- a memory (120) operably connected to the processor and having encoded thereon, computer-executable program code, configured to:
receive tracking data (505) identifying the location and orientation of an ultrasound probe (200) relative to an anatomical landmark (400) during an ultrasound imaging procedure;
receiving interaction data (509) from a log file (132) and identifying keyframes (511) from the interaction data (509);
planning a path (401-404) from the landmark (400) to a position corresponding to each keyframe (511); and
generating the ultrasound self-scan training video showing the landmark (400) and planned path (401-404) to the location corresponding to each keyframe (511).

14. The system of claim 13, further comprising:
- an ultrasound probe (200); and
- a camera (300);
wherein, the probe and the camera are operably connected to the processor (110);
wherein the camera is configured to generate a video stream 501) capturing an anatomical landmark (400) and the ultrasound probe (200) during an initial scan; and
wherein the computer-executable program code is further configured to:
control the ultrasound probe (200) to propagate and receive ultrasound signals and receive ultrasound image data from the ultrasound probe; and
receive the video stream (501) from the camera (300), identify the landmark (400) on the video image stream (501), determine the location of the ultrasound probe (200) relative to the anatomical landmark (400), and provide the location of the ultrasound probe (200) relative to the anatomical landmark (400) as tracking data (505).

15. The system of claim 13, further comprising:
- an ultrasound probe (200); and
- a tracking sensor (195) mounted on the ultrasound probe;
wherein, the probe and the tracking sensor are operably connected to the processor (110); wherein the tracking sensor (195) is configured to generate tracking data capturing locations for an anatomical landmark (400) and the ultrasound probe (200) during an initial scan; and
wherein the computer-executable program code is further configured to:
control the ultrasound probe (200) to propagate and receive ultrasound signals and receive from the ultrasound probe and receive tracking data from the tracking sensor; and
determine the location of the ultrasound probe (200) relative to the anatomical landmark (400).

## Patentansprüche

1. Verfahren zum Erzeugen eines Ultraschall-Selbstscan-Videos, das die folgenden Schritte umfasst:
während eines anfänglichen Ultraschall-Bildgebungsscans, Erfassen (601, 602) einer Stelle eines Orientierungspunkts (400) und von Verfolgungsdaten (505) für eine Ultraschallsonde (200) mit Zeitstempeln (503), und
Benutzerinteraktionen mit Zeitstempeln (507), wobei die Benutzerinteraktionen die Identifizierung (603) eines Schlüsselbilds (511) umfassen;
unter Verwendung der Stelle des Orientierungspunkts (400), der Verfolgungsdaten (505), des identifizierten Schlüsselbilds (511) und der Zeitstempel (503, 507), Planen (604) eines Pfads (401-404) von dem Orientierungspunkt (400) zu einer Stelle der Ultraschallsonde (200), die dem Schlüsselbild (511) entspricht; und
Erzeugen (605) des Ultraschall-Selbstscan-Anweisungsvideos, das den Orientierungspunkt (400) und den geplanten Pfad (401-404) zu der Stelle der Sonde (200) zeigt, die dem Schlüsselbild (511) entspricht.

2. Verfahren nach Anspruch 1, wobei die Benutzerinteraktionen weiter Ultraschallsondeneinstellungen umfassen und das Selbstscan-Trainingsvideo weiter die Ultraschallsondeneinstellungen angibt.

3. Verfahren nach Anspruch 1, wobei das Erfassen (601) einer Stelle eines Orientierungspunkts (400) und von Verfolgungsdaten (505) für eine Ultraschallsonde (200) das Aufzeichnen eines Videos des anfänglichen Ultraschall-Bildgebungsscans und das Extrahieren der Stelle des Orientierungspunks und von Positionsdaten der Ultraschallsonde aus dem Video umfasst.

4. Verfahren nach Anspruch 1, wobei das Erfassen (602) von Benutzerinteraktionen das Extrahieren von Benutzerinteraktionsdaten (509) aus einer Protokolldatei (132) umfasst.

5. Verfahren nach Anspruch 1, wobei das Erfassen (602) von Benutzerinteraktionen das Aufzeichnen eines Videos einer Benutzeroberfläche und das Extrahieren von Benutzerinteraktionen aus dem Video umfasst.

6. Verfahren nach Anspruch 1, wobei der Schritt des Erzeugens (605) des Ultraschall-Selbstscan-Trainingsvideos Videobilder umfasst, die während des anfänglichen Ultraschall-Bildgebungsscans aufgezeichnet wurden.

7. Verfahren nach Anspruch 6, wobei die Videobilder bearbeitet werden, um zu zeigen, wie die Ultraschallsonde (200) dem geplanten Pfad (401-404) von dem Orientierungspunkt (400) zu der Stelle folgt, die dem Schlüsselbild (511) entspricht.

8. Verfahren nach Anspruch 1, wobei:
um jede dem Schlüsselbild (511) entsprechende Stelle herum eine Schlüsselzone (411-414) identifiziert wird, um Verfolgungsungenauigkeiten auszugleichen; und
der Schritt des Erzeugens (605) des Ultraschall-Selbstscan-Trainingsvideos das Erstellen einer Animation einer Ultraschallsonde (200) umfasst, die dem geplanten Pfad (401-404) von dem Orientierungspunkt (400) zu der Stelle folgt, die dem Schlüsselbild (511) entspricht.

9. Verfahren nach Anspruch 2, wobei der Schritt des Erzeugens (605) des Ultraschall-Selbstscan-Trainingsvideos grafische oder akustische Anweisungen umfasst, um die Sondeneinstellungen zu duplizieren.

10. Verfahren nach Anspruch 9, wobei der Schritt des Erzeugens (605) des Ultraschall-Selbstscan-Trainingsvideos weiter das Bereitstellen grafischer oder akustischer Anweisungen umfasst, um einen oder mehrere Übungsdurchgänge des geplanten Pfads (401-404) bei ausgeschaltetem Ultraschallscanner durchzuführen, bevor ein Ultraschall-Selbstscan bei eingeschaltetem Ultraschallscanner durchgeführt wird.

11. Verfahren nach Anspruch 10, wobei der Schritt des Erzeugens (605) des Ultraschall-Selbstscan-Anleitungsvideos weiter umfasst:
Konfigurieren des Ultraschall-Selbstscan-Anleitungsvideos, um Verfolgungsdaten für eine Selbstscan-Ultraschallsonde zu erhalten; und
Überlagern eines Positionsindikators für die Selbstscan-Sonde auf das Selbstscan-Ultraschall-Anleitungsvideo, um dem unerfahrenen Benutzer Rückmeldung zu geben.

12. Computerprogrammprodukt, das ein maschinenlesbares Medium umfasst, auf dem ein maschinenausführbares Programm mit Anweisungen codiert ist, das, wenn es von einem Prozessor (110) ausgeführt wird, die folgenden Schritte durchführt:
während eines anfänglichen Ultraschall-Bildgebungsscans, Erfassen (601, 602) einer Stelle eines Orientierungspunkts (400), von Verfolgungsdaten (505) für eine Ultraschallsonde (200) mit Zeitstempeln (503) und Benutzerinteraktionen mit Zeitstempeln (507), wobei die Benutzerinteraktionen das Identifizieren (603) eines Schlüsselbilds (511) umfassen;
unter Verwendung der Stelle des Orientierungspunkts (400), der Verfolgungsdaten (505), des identifizierten Schlüsselbilds (511) und der Zeitstempel (503, 507), Planen (604) eines Pfads (401-404) von dem Orientierungspunkt (400) zu einer Stelle der Ultraschallsonde (200), die dem Schlüsselbild (511) entspricht; und
Erzeugen (605) eines Ultraschall-Selbstscan-Anweisungsvideos, das den Orientierungspunkt (400) und den geplanten Pfad (401-404) zu der Stelle der Sonde (200) zeigt, die dem Schlüsselbild (511) entspricht.

13. System (100) zum Erzeugen eines Ultraschall-Selbstscan-Trainingsvideos, umfassend:
- einen Prozessor (110); und
- einen Speicher (120), der betriebsfähig mit dem Prozessor verbunden ist und auf dem computerausführbarer Programmcode codiert ist, der dazu konfiguriert ist:
Verfolgungsdaten (505) zu empfangen, die die Stelle und Ausrichtung einer Ultraschallsonde (200) relativ zu einem anatomischen Orientierungspunkt (400) während einer Ultraschallbildgebungsprozedur identifizieren;
Interaktionsdaten (509) aus einer Protokolldatei (132) zu empfangen und Schlüsselbilder (511) anhand der Interaktionsdaten (509) zu identifizieren;
einen Pfad (401-404) von dem Orientierungspunkt (400) zu einer Position, die jedem Schlüsselbild (511) entspricht, zu planen; und
das Ultraschall-Selbstscan-Trainingsvideo, das den Orientierungspunkt (400) und den geplanten Pfad (401-404) zu der Stelle zeigt, die jedem Schlüsselbild (511) entspricht, zu erzeugen.

14. System nach Anspruch 13, weiter umfassend:
- eine Ultraschallsonde (200); und
- eine Kamera (300);
wobei die Sonde und die Kamera betriebsfähig mit dem Prozessor (110) verbunden sind;
wobei die Kamera dazu konfiguriert ist, einen Videostream (501) zu erzeugen, der während eines anfänglichen Scans einen anatomischen Orientierungspunkt (400) und die Ultraschallsonde (200) erfasst; und wobei der computerausführbare Programmcode weiter dazu konfiguriert ist:
die Ultraschallsonde (200) zu steuern, um Ultraschallsignale auszusenden und zu empfangen und Ultraschallbilddaten von der Ultraschallsonde zu empfangen; und
den Videostream (501) von der Kamera (300) zu empfangen, den Orientierungspunkt (400) in dem Videobildstream (501) zu identifizieren, die Stelle der Ultraschallsonde (200) relativ zu dem anatomischen Orientierungspunkt (400) zu bestimmen und die Stelle der Ultraschallsonde (200) relativ zu dem anatomischen Orientierungspunkt (400) als Verfolgungsdaten (505) bereitzustellen.

15. System nach Anspruch 13, weiter umfassend:
- eine Ultraschallsonde (200); und
- einen Verfolgungssensor (195), der an der Ultraschallsonde montiert ist;
wobei die Sonde und der Verfolgungssensor betriebsfähig mit dem Prozessor (110) verbunden sind;
wobei der Verfolgungssensor (195) dazu konfiguriert ist, Verfolgungsdaten zu erzeugen, die Stellen eines anatomischen Orientierungspunkts (400) und der Ultraschallsonde (200) während eines anfänglichen Scans erfassen; und
wobei der computerausführbare Programmcode weiter dazu konfiguriert ist:
die Ultraschallsonde (200) zu steuern, um Ultraschallsignale auszusenden und zu empfangen und von der Ultraschallsonde zu empfangen und Verfolgungsdaten vom Verfolgungssensor zu empfangen; und
die Stelle der Ultraschallsonde (200) relativ zu dem anatomischen Orientierungspunkt (400) zu bestimmen.

## Revendications

1. Procédé de génération d'une vidéo d'auto-échographie, comprenant les étapes de :
capture (601, 602), lors d'un balayage d'imagerie échographique initial, d'un emplacement de point de repère (400) et de données de suivi (505) pour une sonde échographique (200) avec des horodatages (503), et
d'interactions d'utilisateur avec des horodatages (507), dans lequel les interactions d'utilisateur comprennent une identification (603) d'une image clé (511) ;
en utilisant l'emplacement du point de repère (400), les données de suivi (505), l'image clé identifiée (511) et les horodatages (503, 507), planification (604) d'un trajet (401-404) du point de repère (400) à un emplacement de sonde échographique (200) correspondant à l'image clé (511) ; et
génération (605) de la vidéo d'instruction d'auto-échographie montrant le point de repère (400) et le trajet planifié (401-404) vers l'emplacement de sonde (200) correspondant à l'image clé (511).

2. Procédé selon la revendication 1, dans lequel les interactions d'utilisateur comprennent en outre des paramètres de sonde échographique, et la vidéo de formation à l'auto-échographie indique en outre les paramètres de sonde échographique.

3. Procédé selon la revendication 1, dans lequel la capture (601) d'un emplacement du point de repère (400) et de données de suivi (505) pour une sonde échographique (200) comprend l'enregistrement d'une vidéo du balayage d'imagerie échographique initial et l'extraction de l'emplacement de point de repère et des données de position de sonde échographique à partir de la vidéo.

4. Procédé selon la revendication 1, dans lequel la capture (602) d'interactions d'utilisateur comprend l'extraction de données d'interaction d'utilisateur (509) à partir d'un fichier journal (132).

5. Procédé selon la revendication 1, dans lequel la capture (602) des interactions d'utilisateur comprend l'enregistrement d'une vidéo d'une interface utilisateur et l'extraction d'interactions d'utilisateur à partir de la vidéo.

6. Procédé selon la revendication 1, dans lequel l'étape de génération (605) de la vidéo de formation à l'auto-échographie comprend des images vidéo enregistrées pendant le balayage d'imagerie échographique initial.

7. Procédé selon la revendication 6, dans lequel les images vidéo sont éditées pour montrer la sonde échographique (200) suivant le trajet planifié (401-404) du point de repère (400) à l'emplacement correspondant à l'image clé (511).

8. Procédé selon la revendication 1, dans lequel :
une zone clé (411-414) est identifiée autour de chaque emplacement correspondant à l'image clé (511) pour compenser des imprécisions de suivi ; et
l'étape de génération (605) de la vidéo de formation à l'auto-échographie comprend la création d'une animation d'une sonde échographique (200) suivant le trajet planifié (401-404) du point de repère (400) à l'emplacement correspondant à l'image clé (511).

9. Procédé selon la revendication 2, dans lequel l'étape de génération (605) de la vidéo de formation à l'auto-échographie comprend des instructions graphiques ou sonores pour dupliquer les paramètres de sonde.

10. Procédé selon la revendication 9, dans lequel l'étape de génération (605) de la vidéo de formation à l'auto-échographie comprend en outre la fourniture d'instructions graphiques ou sonores pour effectuer un ou plusieurs essais du trajet planifié (401-404) pendant que l'appareil d'échographie est éteint, avant d'effectuer une auto-échographie avec l'appareil d'échographie allumé.

11. Procédé selon la revendication 10, dans lequel l'étape de génération (605) de la vidéo d'instructions d'auto-échographie comprend en outre :
la configuration de la vidéo d'instructions d'auto-échographie pour obtenir des données de suivi pour une sonde d'auto-échographie ; et
la superposition d'un indicateur de position pour la sonde d'auto-échographie sur la vidéo d'instructions d'auto-échographie pour fournir un retour d'information à l'utilisateur non qualifié.

12. Produit programme informatique comprenant un support lisible par machine présentant, codé sur celui-ci, un programme d'instructions exécutable par machine qui, lorsqu'il est exécuté par un processeur (110), exécute les étapes de :
capture (601, 602), lors d'un balayage d'imagerie échographique initial, d'un emplacement de point de repère (400) et de données de suivi (505) pour une sonde échographique (200) avec des horodatages (503), et d'interactions d'utilisateur avec des horodatages (507), dans lequel les interactions d'utilisateur comprennent une identification (603) d'une image clé (511) ;
en utilisant l'emplacement du point de repère (400), les données de suivi (505), l'image clé identifiée (511) et les horodatages (503, 507), planification (604) d'un trajet (401-404) du point de repère (400) à un emplacement de sonde échographique (200) correspondant à l'image clé (511) ; et
génération (605) d'une vidéo d'instruction d'auto-échographie montrant le point de repère (400) et le trajet planifié (401-404) vers l'emplacement de sonde (200) correspondant à l'image clé (511).

13. Système (100) permettant de générer une vidéo de formation à l'auto-échographie, comprenant :
- un processeur (110) ; et
- une mémoire (120) connectée fonctionnellement au processeur et présentant, codé sur celle-ci, un code de programme exécutable par ordinateur, configuré pour :
recevoir des données de suivi (505) identifiant l'emplacement et l'orientation d'une sonde échographique (200) par rapport à un point de repère anatomique (400) pendant une opération d'imagerie échographique ;
recevoir des données d'interaction (509) à partir d'un fichier journal (132) et identifier des images clés (511) à partir des données d'interaction (509) ;
planifier un trajet (401-404) du point de repère (400) à une position correspondant à chaque image clé (511) ; et
générer la vidéo de formation à l'auto-échographie montrant le point de repère (400) et le trajet planifié (401-404) vers l'emplacement correspondant à chaque image clé (511).

14. Système selon la revendication 13, comprenant en outre :
- une sonde échographique (200) ; et
- une caméra (300);
dans lequel la sonde et la caméra sont connectées fonctionnellement au processeur (110) ;
dans lequel la caméra est configurée pour générer un flux vidéo (501) capturant un point de repère anatomique (400) et la sonde échographique (200) lors d'un balayage initial ; et dans lequel le code de programme exécutable par ordinateur est en outre configuré pour :
commander la sonde échographique (200) pour propager et recevoir des signaux échographiques et recevoir des données d'image échographique à partir de la sonde échographique ; et
recevoir le flux vidéo (501) à partir de la caméra (300), identifier le point de repère (400) sur le flux d'images vidéo (501), déterminer l'emplacement de la sonde échographique (200) par rapport au point de repère anatomique (400), et fournir l'emplacement de la sonde échographique (200) par rapport au point de repère anatomique (400) en tant que données de suivi (505).

15. Système selon la revendication 13, comprenant en outre :
- une sonde échographique (200) ; et
- un capteur de suivi (195) monté sur la sonde échographique ;
dans lequel la sonde et le capteur de suivi sont connectés fonctionnellement au processeur (110) ;
dans lequel le capteur de suivi (195) est configuré pour générer des données de suivi capturant des emplacements pour un point de repère anatomique (400) et la sonde échographique (200) pendant un balayage initial ; et
dans lequel le code de programme exécutable par ordinateur est en outre configuré pour :
commander la sonde échographique (200) pour propager et recevoir des signaux échographiques et recevoir à partir de la sonde échographique et recevoir des données de suivi à partir du capteur de suivi ; et
déterminer l'emplacement de la sonde échographique (200) par rapport au point de repère anatomique (400).
